# EUROPEAN PATENT APPLICATION

(11) **EP 1 481 694 A1**
(43) Date of publication of application: **01.12.2004**
(21) Application number: 04253207.7
(22) Date of filing: 28.05.2004
(51) Int. Cl.: A61L 15/28

(54) **Biodegradable hemostatic wound dressings**

(30) Priority: 30.05.2003 US 448878
(71) Applicant: ETHICON, INC., Somerville, NJ 08876 (US)
(72) Inventor: Pendharkar, Sanyog, Old Bridge, NJ 08857 (US); Guo, Jian Xin, Bridgewater, NJ 08807 (US)
(74) Representative: Mercer, Christopher Paul

(57) **Abstract**

The present invention is directed to a hemostatic wound dressing that utilizes a fibrous, fabric substrate made from a biocompatible polymer and containing a first wound-contacting surface and a second surface opposing the first surface, the fabric having flexibility, strength and porosity effective for use as a hemostat; and further having a porous, polymeric matrix distributed at least on the first surface and through the fabric, the porous, polymeric matrix being made of a biodegradable biocompatible, water-soluble or water-swellable aldehyde-oxidized polysaccharide.

## Description

The present invention relates to hemostatic wound dressings containing a fabric substrate and a porous, biocompatible, biodegradable, water-soluble or water-swellable polymeric matrix.

### BACKGROUND OF THE INVENTION

The control of bleeding is essential and critical in surgical procedures to minimize blood loss, to reduce post-surgical complications, and to shorten the duration of the surgery in the operating room. Due to its biodegradability and its bactericidal and hemostatic properties, cellulose that has been oxidized to contain carboxylic acid moieties, hereinafter referred to as carboxylic-oxidized cellulose, has long been used as a topical hemostatic wound dressing in a variety of surgical procedures, including neurosurgery, abdominal surgery, cardiovascular surgery, thoracic surgery, head and neck surgery, pelvic surgery and skin and subcutaneous tissue procedures.

Currently utilized hemostatic wound dressings include knitted or non-woven fabrics comprising carboxylic-oxidized cellulose. Currently utilized oxidized regenerated cellulose is carboxylic-oxidized cellulose comprising reactive carboxylic acid groups and which has been treated to increase homogeneity of the cellulose fiber. Examples of such hemostatic wound dressings commercially available include Surgicel® absorbable hemostat; Surgicel Nu-Knit® absorbable hemostat; and Surgicel® Fibrillar absorbable hemostat; all available from Johnson & Johnson Wound Management Worldwide, a division of Ethicon, Inc., Somerville, New Jersey, a Johnson & Johnson Company. Other examples of commercial absorbable hemostats containing carboxylic-oxidized cellulose include Oxycel® absorbable cellulose surgical dressing from Becton Dickinson and Company, Morris Plains, New Jersey. The oxidized cellulose hemostats noted above are knitted fabrics having a porous structure effective for providing hemostasis. They exhibit good tensile and compressive strength and are flexible such that a physician can effectively place the hemostat in position and maneuver the dressing during the particular procedure being performed.

It would be advantageous to provide hemostatic wound dressing that can be used in internal surgical procedures. For such procedures, bioabsorbability and/or biodegradability of an implant, e.g. a hemostat, is very important, as residual foreign matter may cause undesirable tissue reactions.

The present invention provides biodegradable wound dressings that provide hemostatic and anti-microbial properties equivalent to or better than conventional carboxylic-oxidized cellulose-based hemostatic wound dressings.

### SUMMARY OF THE INVENTION

The present invention is directed to hemostatic wound dressings comprising a fabric substrate, said fabric substrate comprising a first wound-contacting surface and a second surface opposing said first wound-contacting surface, said fabric substrate comprising fibers and having flexibility, strength and porosity effective for use as a hemostat, said fabric comprising a biocompatible polymer; and a porous, polymeric matrix distributed at least on said first wound contacting surface and through said fabric substrate, said porous, polymeric matrix comprising a biocompatible, biodegradable water-soluble or water-swellable aldehyde-oxidized polysaccharide.

### BRIEF DESCRIPTION OF THE FIGURES

Figure 1 is a scanning electron microscopy image (X75) of a cross section of a comparative wound dressing.
Figure 2 is a scanning electron microscopy image (X75) of the first surface of a comparative wound dressing.
Figure 3 is a scanning electron microscopy image (X75) of a cross section of a comparative wound dressing.
Figure 4 is a scanning electron microscopy image (X75) of the first surface of a comparative wound dressing.
Figure 5 is a scanning electron microscopy image (X75) of the second opposing surface of a comparative wound dressing.
Figure 6 is a scanning electron microscopy image (X75) of a cross-section of a wound dressing of the present invention.
Figure 7 is a scanning electron microscopy image (X75) of the first wound-contacting surface of a wound dressing of the present invention.
Figure 8 is a scanning electron microscopy image (X75) of the second opposing surface of a wound dressing of the present invention.
Figure 9 is a scanning electron microscopy image (X75) of a cross-section of a wound dressing of the present invention.
Figure 10 is a scanning electron microscopy image (X75) of the first wound-contacting surface of a wound dressing of the present invention.
Figure 11 is a scanning electron microscopy image (X75) of the second opposing surface of a wound dressing of the present invention.

### DETAILED DESCRIPTION OF THE INVENTION

We have discovered certain hemostatic wound dressings that utilize a fabric as a substrate, where the fabric substrate comprises fibers prepared from a biocompatible polymer, a first wound-contacting surface, a second surface opposing the first surface, and that possesses properties suitable for use as a hemostat, e.g. strength, flexibility and porosity. A more detailed description of such fabric properties is presented herein below. The wound dressings further comprise a biodegradable, porous, polymeric matrix disposed at least on the first wound-contacting surface and distributed through the fabric substrate. Preferably, the porous, polymeric matrix further is disposed on the second opposing surface. The polymeric matrix may be disposed substantially homogenously on the first wound-contacting surface and through the substrate. The second surface also may contact the wound or body tissue when placed on or in the body. The hemostatic wound dressings of the present invention provide and maintain effective hemostasis when applied to a wound requiring hemostasis. Effective hemostasis, as used herein, is the ability to control and/or abate capillary, venous, or arteriole bleeding within an effective time, as recognized by those skilled in the art of hemostasis. Further indications of effective hemostasis may be provided by governmental regulatory standards and the like.

Fabrics utilized in conventional hemostatic wound dressings, such as Surgicel® absorbable hemostat; Surgicel Nu-Knit® absorbable hemostat; and Surgicel® Fibrillar absorbable hemostat; all available from Johnson & Johnson Wound Management Worldwide, a division of Ethicon, Inc., Somerville, New Jersey, a Johnson & Johnson Company, as well as Oxycel® absorbable cellulose surgical dressing from Becton Dickinson and Company, Morris Plains, New Jersey, all may be used in preparing wound dressings according to the present invention.

In certain embodiments, wound dressings of the present invention are effective in providing and maintaining hemostasis in cases of severe bleeding. As used herein, severe bleeding is meant to include those cases of bleeding where a relatively high volume of blood is lost at a relatively high rate. Examples of severe bleeding include, without limitation, bleeding due to arterial puncture, liver resection, blunt liver trauma, blunt spleen trauma, aortic aneurysm, bleeding from patients with over-anticoagulation, or bleeding from patients with coagulopathies, such as hemophilia. Such wound dressings allow a patient to ambulate quicker than the current standard of care following, e.g. a diagnostic or interventional endovascular procedure.

The composite hemostat of the present invention remains very flexible, conforms to a bleeding site and retains good tensile and compressive strength to withstand handling during application. The hemostat can be cut into different sizes and shapes to fit the surgical needs. It can be rolled up or packed into irregular anatomic areas. The fabric in a preferred embodiment capable of providing and maintaining effective hemostasis is a knitted carboxylic-oxidized regenerated cellulose, such as the fabric used to manufacture Surgicel Nu-Knit® absorbable hemostat available from Ethicon, Inc., Somerville, New Jersey.

In certain embodiments of the invention, the wound dressings may further include hemostatic agents, or other biological or therapeutic agents, moieties or species, including drugs and pharmaceutical agents that are acid-insensitive. By acid-insensitive it is meant that they are not detrimentally affected by acid species, such as carboxyl groups contained on conventional carboxylic-oxidized cellulose wound dressings such that they are no longer useful for their intended purpose. The agents may be bound within the polymeric matrix, as well as to the fabric surfaces and/or within the fabric. The agents may be bound by chemical or physical means. The agents may be dispersed partially or homogenously through the fabric and/or the polymeric matrix. Examples of therapeutic agents include, but are not limited to, analgesics, antibiotics, anti-bacterial agents, vaso-constricting agents, anti-adhesion agents, styptic agents and growth factors. One skilled in the art, once having the benefit of the disclosure of the present invention, would have sufficient information such that they would be able to add an acid-insensitive agent to the hemostatic wound dressing described herein.

The fabric substrates utilized in the present invention may be woven or nonwoven, provided that the fabric possesses the physical properties necessary for use in hemostatic wound dressings. A preferred woven fabric has a dense, knitted structure that provides form and shape for the hemostatic wound dressings. Such fabrics are described in US-A-4,626,253.

In preferred embodiments of the present invention, the absorbable hemostatic fabrics are warp knitted tricot fabrics constructed of bright rayon yam which is subsequently oxidized to include carboxyl or aldehyde moieties in amounts effective to provide the fabrics with biodegradability and anti-microbial activity. The fabrics are characterized by having a single ply thickness of at least about 0.5 mm, a density of at least about 0.03 g/cm², air porosity of less than about 150 cm³/sec/cm², and liquid absorption capacity of at least about 3 times the dry weight of the fabric and at least about 0.1 g water per cm² of the fabric.

The knitted fabrics have good bulk without undue weight, are soft and drapable, and conform well to the configuration of the surface to which they are applied. The fabric may be cut into suitable sizes and shapes without running or fraying along the cut edge. Fabric strength after oxidation is adequate for use as a surgical hemostat.

Preferred hemostatic fabrics used in the present invention comprise oxidized cellulose and are best characterized by their physical properties of thickness, bulk, porosity and liquid absorption capacity, as recited above. Suitable fabrics having these properties may be constructed by knitting 60 denier, 18-filament bright rayon yam on a 32-gauge machine at a knit quality of 12. A suitable tricot fabric construction is front-bar 1-0, 10-11; back-bar 2-3, 1-0. The extended shog movement imparted to the front bar results in a 188 inch runner compared to a 70 inch runner for the back guide bar, and increases the fabric bulk and density. The ratio of front to back bar runners in this particular construction is 1:2.7.

Typical physical and hemostatic properties of preferred fabrics produced as described above are noted in Table 1.

**TABLE I**

| Property | |
|---|---|
| Thickness (mm); | 0.645 |
| Density (g/cm²); | 0.052 |
| Air Porosity (cm³/sec/cm²); | 62.8 |
| Tensile Strength⁽¹⁾(md/cd)Kg; | 1.9/4.5 |
| Elongation⁽²⁾ (%); | 23/49 |
| Absorption⁽³⁾ (g/g fabric); | 3.88 |
| (g/cm² fabric); | 0.20 |
| Hemostasis⁽⁴⁾ (min) | |
| 1 ply; | 5.7±1.0 |
| 2 ply; | 5.6±1.8 |

| | |
|---|---|
| ⁽¹⁾ tensile strength determined at 2 in/min extension md/cd = machine direction/cross direction. | |
| ⁽²⁾ Elongation, machine direction/cross direction. | |
| ⁽³⁾ Absorption based on weight of water absorbed by fabric. | |
| ⁽⁴⁾ Hemostasis evaluation on incised porcine splenic wounds, time to stop bleeding. | |

The tricot fabrics utilized in the present invention may be constructed from bright rayon yams of from about 40 to 80 total denier. Each yam may contain from 10 to 25 individual filaments, although each individual filament preferably is less than 5 denier to avoid extended absorption times. The high bulk and fabric density are obtained by knitting at 28 gauge or finer, preferably at 32 gauge, with a fabric quality of about 10 or 12 (40 to 48 courses per inch). A long guide bar shog movement of at least 6 needle spaces, and preferably 8 to 12 spaces, further increases fabric thickness and density.

Other warp knit tricot fabric constructions which produce equivalent physical properties may, of course, be utilized in the manufacture of the improved hemostatic fabrics and wound dressings of the present invention, and such constructions will be apparent to those skilled in the art.

Polymers useful in preparing the fabric substrates in wound dressings of the present invention include, without limitation, collagen, calcium alginate, chitin, polyester, polypropylene, polysaccharides, polyacrylic acids, polymethacrylic acids, polyamines, polyimines, polyamides, polyesters, polyethers, polynucleotides, polynucleic acids, polypeptides, proteins, poly (alkylene oxide), polyalkylenes, polythioesters, polythioethers, polyvinyls, polymers comprising lipids, and mixtures thereof.

Preferably, carboxylic-oxidized polysaccharides are used to prepare wound dressings of the present invention. More preferably, carboxylic-oxizided cellulose is used to prepare fabrics used in wound dressings of the present invention. Even more preferably, carboxylic-oxidized regenerated cellulose is used to prepare fabric substrates used in wound dressings of the present invention. Regenerated cellulose is preferred due to its higher degree of uniformity versus cellulose that has not been regenerated. Regenerated cellulose and a detailed description of how to make regenerated carboxylic-oxidized cellulose is set forth in US-A-3,364,200 and US-A-5,180,398. As such, teachings concerning regenerated oxidized cellulose and methods of making same are well within the knowledge of one skilled in the art of hemostatic wound dressings.

Certain of the wound dressings of the present invention utilize fabric substrates that have been oxidized to contain carboxyl moieties in amounts effective to provide the fabrics with biodegradability and anti-microbial activity. US-A-3,364,200 discloses the preparation of carboxylic-oxidized cellulose with an oxidizing agent such as dinitrogen tetroxide in a Freon medium. US-A-5,180,398 discloses the preparation of carboxylic-oxidized cellulose with an oxidizing agent such as nitrogen dioxide in a per-fluorocarbon solvent. After oxidation by either method, the fabric is thoroughly washed with a solvent such as carbon tetrachloride, followed by aqueous solution of 50 percent isopropyl alcohol (IPA), and finally with 99% IPA. Prior to oxidation, the fabric is constructed in the desired woven or nonwoven construct suitable for use as a hemostat. Certain wound dressings according to the present invention that utilize such fabrics have been found to provide and maintain hemostasis in cases of severe bleeding.

It has been found that the fabric preferably is conditioned prior to saturation with polymer solution and lyophilization as described herein in order to provide substantially homogenous distribution of the polymer solution on and through the fabric substrate. Conditioning of the fabric can be achieved by storing the fabric at room temperature under ambient conditions for at least 6 month, or conditioning of the fabric can be accelerated. Preferably, the fabric is exposed to conditions of about 4°C to about 90°C, at a relative humidity of from about 5% to about 90%, for a time of from about 1 hour to 48 months. More preferably, the fabric is exposed to conditions of about 4°C to about 60°C, at a relative humidity of from about 30% to about 90%, for a time of from about 72 hours to 48 months. Even more preferably, the fabric is exposed to conditions of about 18°C to about 50°C, at a relative humidity of from about 60% to about 80%, for a time of from about 72 hours to 366 hours. Most preferably, the fabric is conditioned at a temperature of about 50°C, at a relative humidity of about 70%, for a time of about 168 hours. The fabric may be placed horizontally in a conditioned environment, taking care to provide spacing between the fabric substrates to allow proper conditioning. The fabric also may be suspended vertically to allow conditioning.

As result of the conditioning of the carboxylic-oxidized cellulose fabric substrate, the fabric substrate will comprise at least about 3 weight percent of water-soluble molecules, preferably from about 3 to about 30 weight percent, more preferably from about 8 to about 20 weight percent, even more preferably from about 9 to about 12 weight percent, and most preferably about 10 weight percent. In general, the water-soluble molecules are acid-substituted oligosaccharides containing approximately 5 or fewer saccharide rings. It has been found that the hemostatic efficacy of the wound dressing containing such carboxylic-oxidized cellulose fabric substrates, including the occurrence of re-bleeding of a wound for which hemostasis initially has been achieved, is improved when the contents of the water-soluble molecules reach about 8%, preferably about 10%, based on the weight of the fabric substrate.

Fabric substrates used in the present invention also will comprise from about 3 to about 20 weight percent of water, preferably from about 7 to about 13 weight percent, and more preferably from about 9 to about 12 weight percent water.

Similar levels of moisture and water-soluble molecules in the carboxylic-oxidized cellulose fabric substrate also may be achieved by other means. For example, sterilization of the fabric by known techniques, such as gamma or e-beam irradiation, may provide similar content of water and/or water-soluble molecules. In addition, water-soluble molecules such as oligosaccharides could be added to the fabric prior to distribution of the porous, polymeric matrix on and through the fabric. Once having the benefit of this disclosure, those skilled in the art may readily ascertain other methods for providing such fabrics with moisture and/or water-soluble molecules.

As noted above, wound dressings of the present invention comprise a biodegradable, porous, polymeric matrix disposed at least on the first wound-contacting surface and dispersed through the fabric substrate. Preferably, the matrix also is disposed on the second opposing surface. The polymer matrix may be homogenously disposed on both of the first and second surfaces and dispersed through the fabric. The polymer used to prepare the porous, polymeric matrix in wound dressings of the present invention is a biocompatible, biodegradable water-soluble or water-swellable aldehyde-oxidized polysaccharide. The water-soluble or water-swellable polysaccharide rapidly absorbs blood or other body fluids and forms a tacky or sticky gel adhered to tissue when placed in contact therewith. The fluid-absorbing polysaccharide, when in a dry or concentrated state, interacts with body fluid through a hydration process. Once applied in a bleeding site, the polysaccharide interacts with the water component in the blood via the hydration process. The hydration force provides an adhesive interaction that aids the hemostat adhere to the bleeding site. The adhesion creates a sealing layer between the hemostat and the bleeding site to stop the blood flow.

Polysaccharides that may be aldehyde-oxidized according to the present invention include, without limitation, cellulose, cellulose derivatives, e.g. alkyl cellulose, for instance methyl cellulose, hydroxyalkyl cellulose, alkylhydroxyalkyl cellulose, cellulose sulfate, salts of carboxymethyl cellulose, carboxymethyl cellulose and carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid and mixtures and derivatives of the above.

In such wound dressings, the aldehyde-oxidized polysaccharide will contain an amount of aldehyde moieties effective to render the modified polysaccharide biodegradable, meaning that the polysaccharide is degradable by the body into components that either are resorbable by the body, or that can be passed readily by the body. More particularly, the biodegraded components do not elicit permanent chronic foreign body reaction when they are absorbed by the body, such that no permanent trace or residual of the component is retained at the implantation site.

In preferred embodiments utilizing aldehyde-oxidized polysaccharides, the polysaccharide is oxidized as described herein to assure that the aldehyde-oxidized polysaccharide is biodegradable. Such biodegradable, aldehyde-oxidized polysaccharides may be represented by Structure I below. 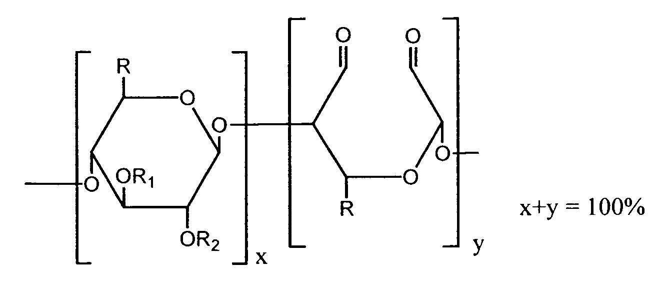 where x and y represent mole percent, x plus y equals 100 percent, x is from about 95 to about 5, y is from about 5 to about 95; and R may be CH₂OR₃, COOR₄, sulphonic acid, or phosphonic acid; R₃ and R₄ may be H, alkyl, aryl, alkoxy or aryloxy, and R₁ and R₂ may be H, alkyl, aryl, alkoxy, aryloxy, sulphonyl or phosphoryl.

One method of making the wound dressings of the inventions is set forth below. The steps involved in the preparation of the novel porous structure comprise dissolving the appropriate polymer to be lyophilized in an appropriate solvent for the polymer to prepare a homogenous polymer solution. The fabric then is contacted with the polymer solution such that it is saturated with the polymer solution. The fabric substrate and polymer solution incorporated in the dense construct of the fabric then is subjected to a freezing and vacuum drying cycle. The freezing/drying step phase removes the solvent by sublimation, leaving a porous, polymer matrix structure disposed on and through the fabric substrate. Through this preferred lyophilization method, the wound dressing comprising a fabric substrate that comprises a matrix of the water-soluble or water-swellable polymer and having microporous and/or nanoporous structure is obtained. The lyophilization conditions are important to the novel porous structure in order to create a large matrix surface area in the hemostat with which body fluids can interact once the dressing is applied to a wound requiring hemostasis.

During the lyophilization process, several parameters and procedures are important to produce wound dressings having mechanical properties suitable for use in hemostatic wound dressings. The features of such microporous structure can be controlled to suit a desired application by choosing the appropriate conditions to form the composite hemostat during lyophilization. The type of microporous morphology developed during the lyophilization is a function of such factors, such as the solution thermodynamics, freezing rate, temperature to which it is frozen, and concentration of the solution. To maximize the surface area of the porous matrix of the present invention, a preferred method is to quickly freeze the fabric/polymer construct at lower than 0°C, preferably at about -50°C, and to remove the solvent under high vacuum. The porous matrix produced thereby provides a large fluid-absorbing capacity to the hemostatic wound dressing. When the hemostatic wound dressing comes into contact with body fluid, a very large surface area of polymer is exposed to the fluid instantly. The hydration force of the hemostat and subsequent formation of a tacky gelatinous layer helps to create an adhesive interaction between the hemostat and the bleeding site. The microporous structure of the polymeric matrix also allows blood to quickly pass through the fabric surface before the hydration takes place, thus providing an increased amount of the polymer to come in contact with the body fluids. The formation of a gelatinous sheet on oxidized cellulose upon blood contact will enhance the sealing property of the water-soluble gelatinous layer, which is critical to rapid hemostasis in cases ranging from moderate to severe bleeding.

The fabric substrate comprises the biodegradable polymeric matrix in an amount effective to provide and maintain effective hemostasis; in some embodiments in cases of severe bleeding. If the ratio of polymer to fabric is too low, the polymer does not provide an effective seal to physically block the bleeding, thus reducing the hemostatic properties. If the ratio is too high, the composite hemostat wound dressing will be too stiff or too brittle to conform to wound tissue in surgical applications, thus adversely affecting the mechanical properties necessary for handling by the physician in placement and manipulation of the dressing. Such an excessive ratio will also prevent the blood from quickly passing through the fabric surface to form the gelatinous layer on the oxidized biodegradable cellulose that is critical for enhancing the sealing property. A preferred weight ratio of polymer to fabric is from about 1:99 to about 15:85. A more preferred weight ratio of polymer to fabric is from about 3:97 to about 10:90.

Wound dressings of the present invention are best exemplified in the figures prepared by scanning electron microscope. The samples were prepared by cutting 1-cm² sections of the dressings by using a razor. Micrographs of both the first surface and opposing second surface, and cross-sections were prepared and mounted on carbon stubs using carbon paint. The samples were gold-sputtered and examined by scanning electron microscopy (SEM) under high vacuum at 4KV.

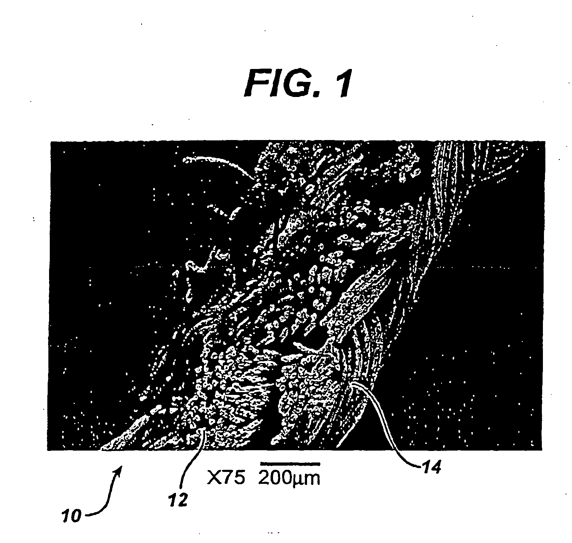

Figure 1 is a cross-section view (75X) of uncoated carboxylic-oxidized regenerated cellulose fibers 12 organized as fiber bundles 14 and knitted into fabric 10 according to preferred embodiments of the invention discussed herein above. One commercial example of such a fabric is Surgicel Nu-Knit® absorbable hemostatic wound dressing.

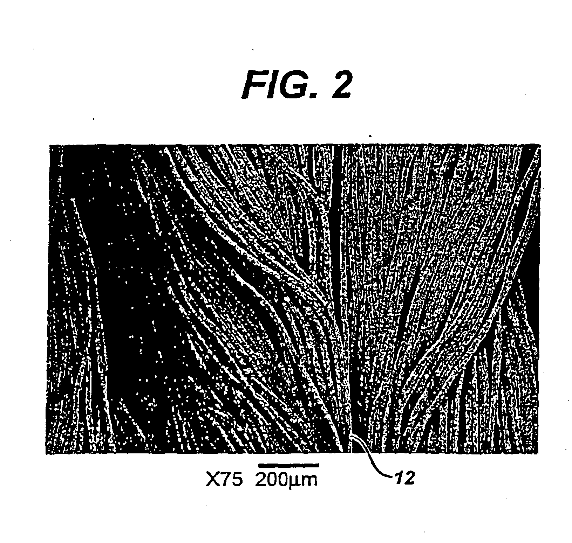

Figure 2 is a view of a first surface of the fabric of Figure 1. Individual fibers 12 are shown within a bundle.

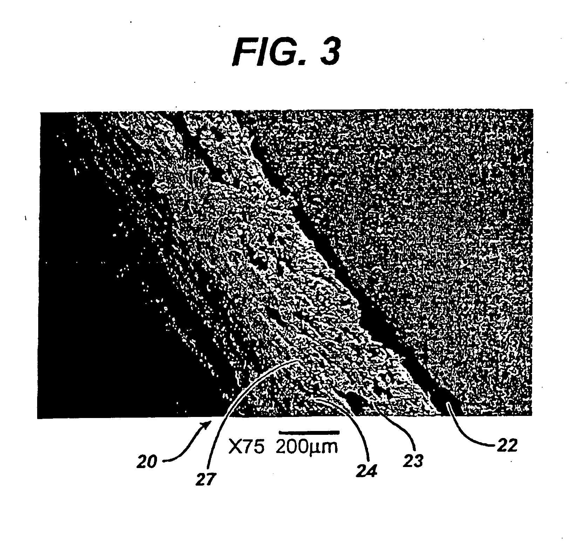

Figure 3 is a cross-section view of fabric 20 having first wound-contacting surface 22 and opposing surface 24 and that has been saturated with a solution of polymer and then air dried, i.e. without lyophilization. Individual fibers 23 also are shown.

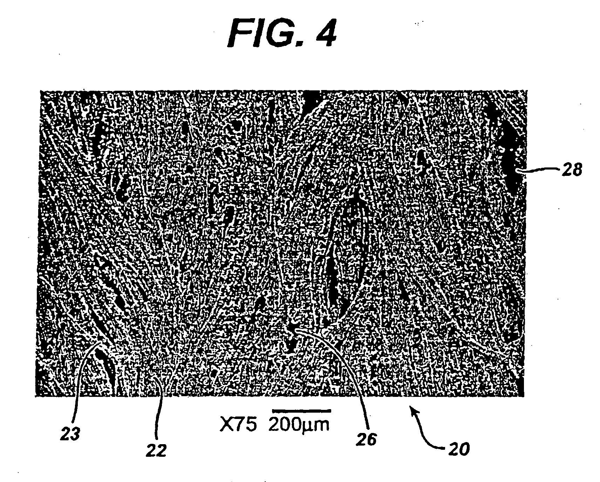

Figure 4 is a view of surface 22 of fabric 20. As observed therein, in the course of air-drying, polymer 26 agglomerates and adheres to fibers 23, in many instances adhering fibers 23 one to the other and creating large voids 28 in the hemostatic fabric through which body fluids may pass. Polymer 26 dispersed on and through fabric 20 is not in the state of a porous matrix and thus provides no hemostasis in cases of severe bleeding as described herein above due, at least in part, to a lack of sufficient porosity, e.g. surface area, to provide polymer/body fluid interaction effective to provide and maintain hemostasis in cases of severe bleeding.

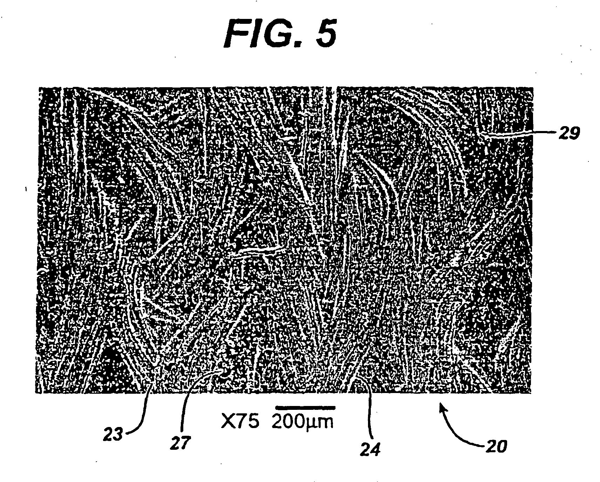

Figure 5 is a view of opposing surface 24 of fabric 20. As shown, opposing surface 24 contains a larger concentration of polymer coating material as opposed to surface 22 shown in Figure 4, obscuring most of fibers 23, although the knitting pattern could still be discerned. The coating was thick enough to span across all of the fibers and generate an intact layer 27 of its own, also shown in Figure 3. This layer appeared to be brittle, as cracks 29 in the coating were observed. The coating layer thickness varied from as thin as about 3 microns in some sections to about 30-65 microns in other sections.

In comparing the surface morphologies of surface 22 and opposing surface 24 of fabric 20, it is apparent that surface 22 contained significantly less polymer. The coating was significantly thinner on the fibers than the coating on the opposing surface. While some polymer was observed to span across some fibers, the coating was incomplete or had perforations present. The coating layer thickness, where present, did not exceed about 2 microns.

It is clear from Figures 3-5 that the fabrics prepared by air-drying do not contain a porous, polymeric matrix homogenously dispersed on the surfaces and there through. Such fabrics are brittle, stiff, do not conform to wound sites, are not able to be handled by physicians, and generally are not suitable for use as wound dressings.

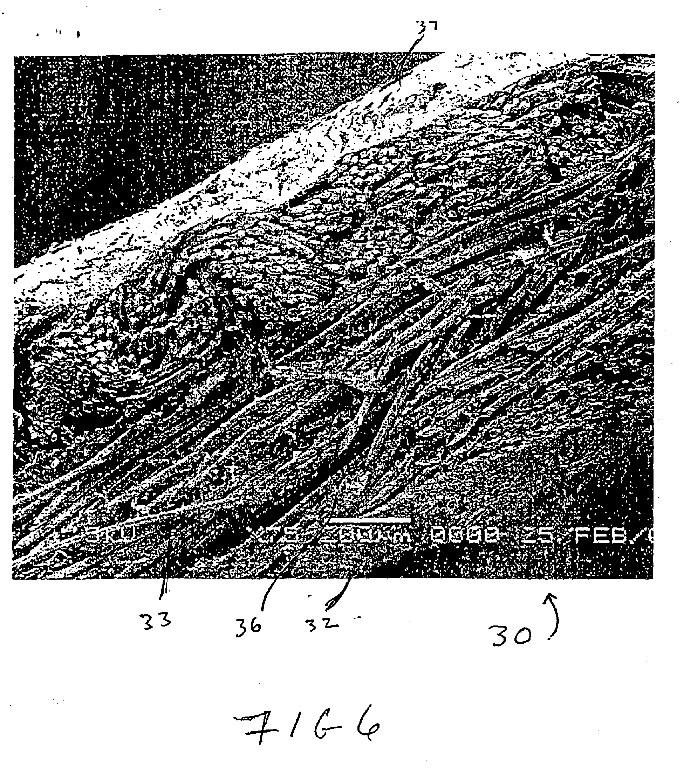

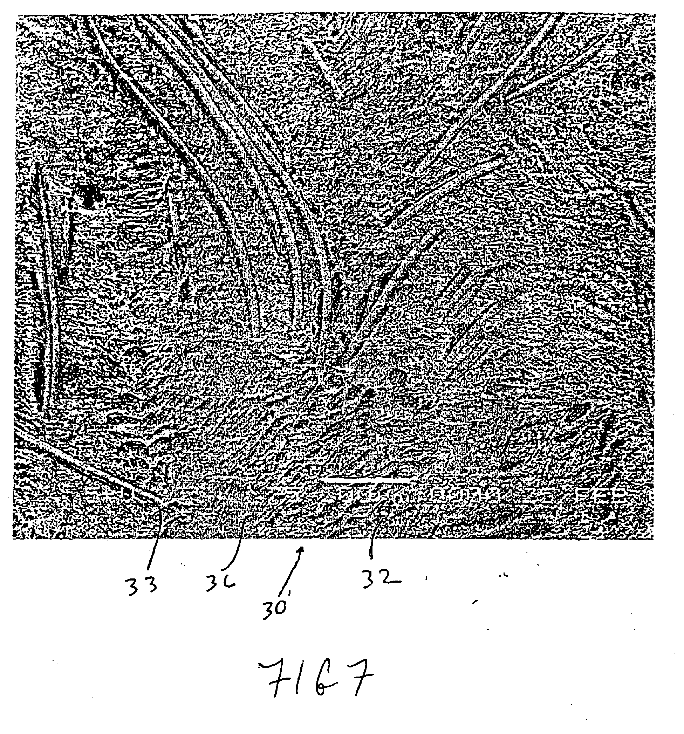

Hemostatic fabrics according to the present invention and comprising aldehyde-oxidized methylcellulose as the polymer matrix are set forth in Figures 6-8. As shown in Figures 6 and 7, a porous, polymer matrix is distributed on wound-contacting surface 32, opposing surface 34 and throughout fabric 30. Polymer 36 forms a porous matrix integrated with knitted fibers 33. The porous polymer matrix exhibits significant liquid absorption properties from capillary action in the same manner as a sponge.

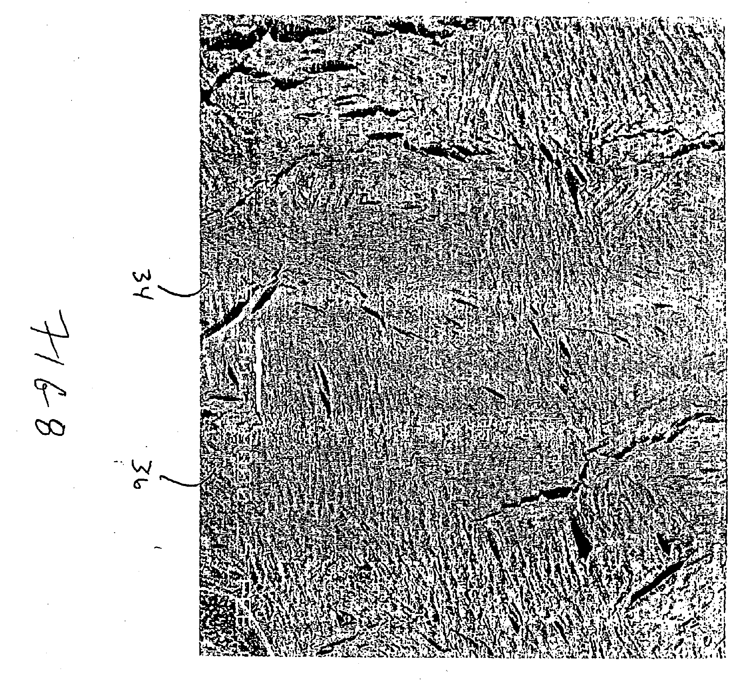

As shown in Figure 7, the polymer matrix disposed on surface 32 contains countless pores, ranging from about two microns to as large as about 35 microns in diameter or greater. As noted, polymer 36 is present in the form of a porous matrix about fibers 33, thereby providing ample polymer surface area with which body fluids can interact upon contact therewith. Opposing surface 34 shown in Figure 8 also contains polymer 36 in the form of a porous matrix.

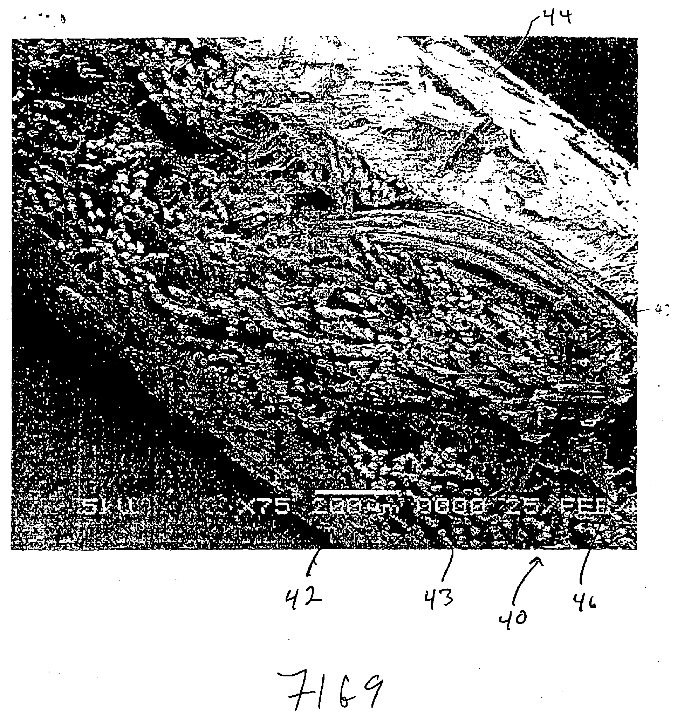

A hemostatic wound dressing fabricated from carboxylic-oxidized regenerated cellulose fabric and a biodegradable polymeric aldehyde-oxidized hydroxyethyl cellulose according to the present invention is represented in Figures 9-11.

As shown in Figure 9, a porous, polymer matrix is distributed on surfaces 42 and 44 and throughout fabric 40. Polymer 46 forms a porous, polymer matrix integrated with the knitted fibers 43. The porous, polymer matrix exhibits significant liquid absorption properties from capillary action in the same manner as a sponge.

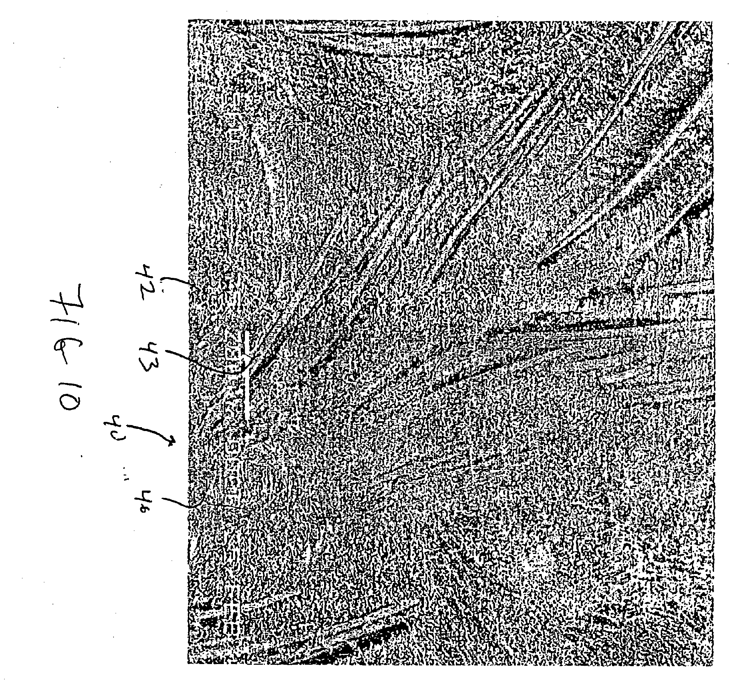

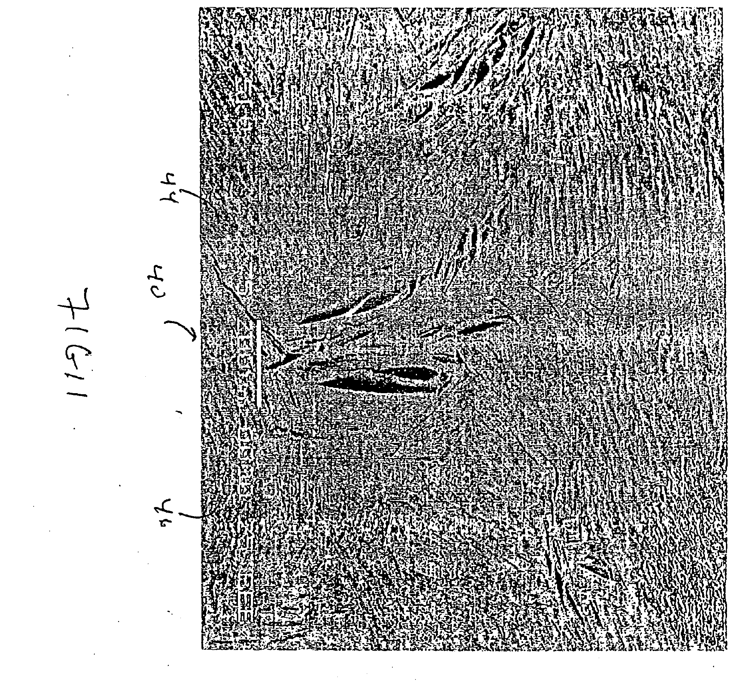

As shown in Figures 10 and 11, the polymer matrix disposed on relative surfaces 42 and 44 contains countless pores, ranging from about ten microns to as large as about 400 microns in diameter, or greater. Figure 10 shows surface 42 of fabric 40. As noted, polymer 46 is present in the form of a porous matrix about fibers 43, thereby providing ample polymer surface area with which body fluids can interact upon contact therewith. Opposing surface 44 shown in Figure 11 also contains polymer 46 in the form of a porous matrix.

It is clear from Figures 6-11 that fabrics and wound dressings of the present invention contain a porous, polymeric matrix dispersed on the surfaces and substantially through the fabric. Due to the porous nature of the matrix, body fluids are permitted to pass into the matrix, where ample surface area of polymer is present to interact with the body fluids. This results in faster and a higher degree of hemostasis.

It also is clear from Figures 3-5 that comparative fabrics and wound dressings do not contain a porous, polymeric matrix, either on a surface of the dressing or dispersed throughout the fabric. As a result, the amount of polymer present to interact with body fluids is significantly reduced. In addition, due to the formation of agglomerated polymer layers during air drying, body fluids are not permitted to pass freely into the wound dressing where they can interact with and bind to the dressing. Additionally, such fabrics were found to be brittle and stiff, such that placement within and conformance to a wound site by a physician is not acceptable.

As stated above, in order to preserve the porous structure of the polymeric matrix and the homogeneity thereof, it is important to maintain the preferred weight ratio of polymer to fabric during the process of making the wound dressing and the homogenous distribution of the polymer solution on the surface of and throughout the fabric substrate in order to avoid defects on and throughout the wound dressing.

In a laboratory setting, this is readily achieved, as the contacting of the fabric substrate with the polymer solution in the laboratory crystallization dish, saturation of the fabric substrate material in the polymer solution and the subsequent lyophilization of the fabric and solution in the dish all take place in the lyophilization unit, where a precise quantity of water-soluble and water-swellable polymer can be used to prepare the solution. No transfer of the saturated fabric or the dish into the lyophilization unit is necessary. As a result, a homogeneous distribution of polymer on fabric is achieved. However, in a manufacturing setting, due to its larger scale, such a process is no longer feasible. A larger container, e.g. a tray or pan, is used instead to hold the fabric and the polymer solution during contacting and saturation of the fabric by the solution, which is conducted outside of the lyophilization unit. The saturated fabric then must be transferred into the lyophilization unit for further processing.

Certain problems are associated with producing a wound dressing of the present invention on a larger scale as described above, where the wound dressing will possess mechanical and hemostatic properties suitable for use as a hemostatic dressing. For instance, if one were to attempt to transfer the container having the polymer solution and saturated fabric disposed therein into the lyophilization unit, during transfer of the container into the lyophilization unit, it is difficult to maintain a constant level of the polymer solution above the fabric in the tray due to movement, e.g. shifting or "sloshing", of the solution in relation to the fabric. In some cases during movement of the container, the fabric surface may even be exposed and the turbulence of the shifting polymer solution in the container may result in poor distribution of polymer on the surface of and through the fabric, particularly with respect to the distribution of the polymer on the surface. This in turn is detrimental to the effectiveness of the hemostatic property of the wound dressing.

In order to maintain the level of solution above the fabric surface prior to lyophilization to ensure that the fabric remains immersed in the polymer solution in order to provide homogeneous distribution on and throughout the fabric, an excess amount of polymer solution must be placed in the container. However, such an approach has not been successful because such an excess amount of polymer solution may result in an undesirable weight ratio of polymer to fabric, which consequently leads to a loss in the flexibility of the wound dressing and of the microporous structure of the polymeric matrix of the hemostatic wound dressing.

To solve this problem, processes of the present invention utilize a transfer support means, e.g. a transfer sheet or carrier, in order to transfer the saturated fabric from the container used to saturate the fabric with polymer solution into the lyophilization unit. However, in order to maintain the homogeneous distribution of the porous, polymeric matrix on and through the fabric substrate after lyophilization, caution must be exercised to minimize disturbance of the homogenous distribution of polymer solution in relationship to the fabric and to minimize deformation, e.g. stretching or tearing, of the fabric substrate during transfer into the lyophilization unit. In addition, the formation of air bubbles or voids between the fabric substrate and the transfer support means while transferring the fabric to the support means must be substantially avoided so as not to create an unacceptable number defects in the wound dressing.

In accordance with the invention, the method provides for the transfer of the saturated fabric substrate onto the transfer support mean and transferring the saturated fabric substrate and support means to a lyophilization unit.

The transfer of the saturated fabric onto the support means is accomplished in a fashion to create a hydraulic pressure sufficient to allow air bubbles to escape from between the fabric substrate and the support means. The distal end of the fabric is joined with the support means and moved in a continuous fashion, at a controlled rate, while maintaining a desired angle of incidence between the fabric and the support means until the proximal end also is supported by the support means to prevent, or at least minimize bubble formation. At the same time, maintaining such conditions of transfer also prevent, or at least minimize, physical deformation of the substrate, such as stretching or tearing. Preferably, the angle of incidence between the fabric and the support means will range from about 20° to about 90°. More preferably, the angle of incidence will range from about 30° to about 60°. Even more preferably, the angle of incidence will be about 45°. The rate of advancing the fabric onto the support means preferably will range from about 8 inches per minute to about 2 inches per minute. Preferably, the rate of transfer is about 7 inches per minute.

The support means should be made of an inert material that will not release any toxic chemical substance or any substance that may alter the characteristics of the wound dressing. It is important that the support means does not alter the freezing and drying parameters associated with the lyophilization process stated above. Therefore, the material used for the support means should be cryolitically stable, such that it may withstand extremely low temperatures without deformation, preferably down to about -50°C. If the support means is not stable at low temperatures, deformation of the means will lead to defects in the wound dressing.

It is important that the support means used for transferring the saturated fabric is of density, mechanical strength, flexibility and thickness to provide sufficient support for the fabric, while avoiding excessive bending and mechanical deformation of the saturated fabric substrate. If the support means is too thick and rigid, it may be difficult to slide the saturated fabric onto the support means. If the supporting means is too soft and flexible, the saturated fabric may bend excessively, thereby causing stretching or other mechanical deformation, which may lead to pooling and running of the polymer solution on the surface, or the creation of surface defects during lyophilization.

It is also important that the support means used for transferring the fabric substrate provides a smooth and flat surface to prevent air bubbles from being trapped under the saturated fabric substrate. The transfer means further must possess heat transfer efficiency suitable for rapid freezing of the fabric/polymer construct and removal of the solvent under high vacuum in the lyophilization unit so as to maintain the homogenous distribution of the porous, polymer matrix on and through the fabric substrate after lyophilization. By heat transfer efficiency, it is meant that heat is transferred quickly from the support means to the saturated fabric to facilitate rapid freezing. A preferred support means is a high-density polyethylene sheet having a preferred thickness of between about 50 mils and about 200 mils. Most preferred support means is high-density polyethylene having a preferred thickness of between about 60 mils and about 100 mils.

While the following examples demonstrate certain embodiments of the invention, they are not to be interpreted as limiting the scope of the invention, but rather as contributing to a complete description of the invention.

### Example 1:

### Preparation of water-soluble aldehyde-oxidized methylcellulose (AOMC):

100 g of a 5% methylcellulose (MC, Ave. Mn 14kD, lot# 13517LO from Aldrich, Milwaukee, WI) aqueous solution was combined with 3 g of periodic acid (Aldrich, Milwaukee, 53201) and was then stirred for 5 hours at ambient temperature in the dark. 1.5 ml of ethylene glycol was added to the reaction solution and stirred for 30 minutes. 2000 ml of acetone were added slowly into the reaction solution to precipitate the aldehyde-oxidized methylcellulose (AOMC). The reaction mixture was allowed to stand for 20-30 minutes to separate the liquid phase from the solid phase. The supernatant then was removed and the solid phase centrifuged to precipitate the solids. The solid precipitate was dissolved in 100 ml DI over night followed by dialysis for 72 hours. The final wet mixture was lyophilized to form a sponge/foam.

### Example 2:

### Preparation of water-soluble aldehyde-oxidized hydroxyethyl cellulose (AOHEC):

100 g of a 5% hydroxyethyl cellulose (HEC, Ave. Mv; 90kD lot # 04220MS from Aldrich, Milwaukee, WI) aqueous solution was combined with 3 g of periodic acid (Aldrich, Milwaukee, 53201) and was then stirred for 5 hours at ambient temperature in the dark. 1.5 ml of ethylene glycol was added to the reaction solution and stirred for 30 minutes. 2000 ml of acetone were added slowly into the reaction solution to precipitate the aldehyde-oxidized hydroxyethyl cellulose. The reaction mixture was allowed to stand for 20-30 minutes to separate the liquid phase from the solid phase. The supernatant then was removed and the solid phase centrifuged to precipitate the solids. The solid precipitate was dissolved in 100 ml DI over night followed by dialysis for 72 hours. The final wet mixture was lyophilized to form a sponge/foam.

### Example 3:

### CORC/HEC porous patch preparation:

2 g of hydroxyethyl cellulose (HEC, Ave. MW 90kD from Aldrich) were dissolved in 98 g of deionized water. After complete dissolution of the polymer, 10 g of the HEC solution was transferred into a crystallization dish with a diameter of 10 cm. A piece of Surgicel Nu-Knit® absorbable hemostat, based on carboxylic oxidized regenerated cellulose (CORC), having a diameter of 9.8 cm (about 1.3 gram) was placed on the HEC solution in the crystallization dish. After soaking the fabric in the solution for 3 minutes, the wet fabric in the dish was then lyophilized overnight. A very flexible patch was formed. The patch was further dried at room temperature under vacuum. The ORC/HEC patch then was evaluated for hemostasis as set forth below. Results are provided in Table 2.

### Example 4:

### CORC/MC porous patch preparation

2 g of methylcellulose (MC, from Aldrich) was dissolved in 98 g of deionized water. After complete dissolution of the polymer, 10 g of the MC solution was transferred into a crystallization dish with a diameter of 10 cm. A piece of Surgicel Nu-Knit® fabric with a diameter of 9.8 cm (about 1.3 gram) was placed on the MC solution in the crystallization dish. After soaking the fabric for 3 minutes, the wet fabric in the dish was then lyophilized overnight. A very flexible patch was formed. The patch was further dried at room temperature under vacuum. The CORC/MC patch then was evaluated for hemostasis as set forth below. Results are provided in Table 1.

### Example 5:

### CORC/AOHEC porous patch preparation:

2 g of aldehyde-oxidized hydroxyethyl cellulose (AOHEC) were dissolved in 98 g of deionized water. After complete dissolution of the polymer, 10 g of the AOHEC solution was transferred into a crystallization dish with a diameter of 10 cm. A piece of Surgicel Nu-Knit® absorbable hemostat, based on carboxylic oxidized regenerated cellulose (CORC), having a diameter of 9.8 cm (about 1.3 gram) was placed on the AOHEC solution in the crystallization dish. After soaking the fabric in the solution for 3 minutes, the wet fabric in the dish was then lyophilized overnight. A very flexible patch was formed. The patch was further dried at room temperature under vacuum. The CORC/AOHEC patch then was evaluated for hemostasis as set forth below. Results are provided in Table 2.

### Example 6:

### CORC/AOMC porous patch preparation

2 g of aldehyde-oxidized methylcellulose (AOMC) was dissolved in 98 g of deionized water. After complete dissolution of the polymer, 10 g of the AOMC solution was transferred into a crystallization dish with a diameter of 10 cm. A piece of Surgicel Nu-Knit® fabric with a diameter of 9.8 cm (about 1.3 gram) was placed on the AOMC solution in the crystallization dish. After soaking the fabric for 3 minutes, the wet fabric in the dish was then lyophilized overnight. A very flexible patch was formed. The patch was further dried at room temperature under vacuum. The ORC/AOMC patch then was evaluated for hemostasis as set forth below. Results are provided in Table 2.

### Example 7:

### Biodegradation Test of Different Materials

1 g samples from Example 3 and 5 were incubated in 100 ml PBS (phosphate buffered saline) buffer (pH 7.2) at 37 °C. A piece of Surgicel Nu-Knit® fabric was used in a similar fashion as the control article. After 48-72 hours clear pale yellow fluids were obtained. Size Exclusion Chromatography was performed on the solutions to assess the molecular weight profile. The experiment was performed in duplicate. A TosohBiosep TSK G5000PWxl SEC column and RI Detector at 40°C were used with a 0.15M NaNO₃ eluent. Polysaccharide standards were used for calibration. For the series, the solution from Example 3 had higher molecular weight soluble polymers than the solution from Example 5, which had more of higher molecular weight soluble polymers than the solution from the Surgicel Nu-Knit® fabric control. This indicated that there was degradation of material from Example 5 in the buffer.

### Example 8:

### Hemostatic performance of different materials in porcine splenic incision model

A porcine spleen incision model was used for hemostasis evaluation of different materials. The materials were cut into 2.5 cm X 1.5 cm rectangles. A linear incision of 1.5 cm with a depth of 0.3 cm was made with a surgical blade on a porcine spleen. After application of the test article, digital tamponade was applied to the incision for 2 minutes. The hemostasis was then evaluated. Additional applications of digital tamponade for 30 seconds each time were used until complete hemostasis was achieved. Fabrics failing to provide hemostasis within 12 minutes were considered to be failures. Table 2 lists the results of the evaluation.

**Table 2**

| Hemostatic performance of Different Materials | |
|---|---|
| Sample | Time to Hemostasis (Minutes) |
| Surgicel Nu-Knit® | 4:00 (n=2) |
| Example 3 | 2:00 (n=2) |
| Example 4 | 2:30 (n=2) |
| Example 5 | 2:00 (n=2) |
| Example 6 | 2:00 (n=2) |
| Surgical gauze Negative Control | >12.00 |

As indicated from the results, wound dressings of the present invention achieve comparable effective hemostasis compared to currently utilized hemostatic wound dressings, showing that imparting biodegradability does not detrimentally alter the hemostatic efficacy.

## Claims

1. A hemostatic wound dressing, comprising:
a fabric substrate, said fabric substrate comprising a first wound-contacting surface and a second surface opposing said first surface, said fabric comprising fibers and having properties effective for use as a hemostat, said fabric comprising a biocompatible polymer; and
a porous, polymeric matrix distributed at least on said first wound-contacting surface and through said fabric, said porous, polymeric matrix comprising a biocompatible, biodegradable water-soluble or water-swellable aldehyde-oxidized polysaccharide.

2. The wound dressing of claim 1 wherein said fabric is woven or non-woven.

3. The wound dressing of claim 1 wherein said fabric is knitted.

4. The wound dressing of any one of claims 1 to 3 wherein said fabric comprises carboxylic-oxidized regenerated cellulose.

5. The wound dressing of any one of claims 1 to 5 wherein said aldehyde-oxidized polysaccharide is prepared from a cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, heparin, heparin sulfate, heparan, heparan sulfate, dermatan sulfate, keratin sulfate, carrageenans, chitosan, starch, amylose, amylopectin, poly-N-glucosamine, polymannuronic acid, polyglucuronic acid, polyguluronic acid or a derivative of the above.

6. The wound dressing of claim 5 wherein said polysaccharide comprises alkyl cellulose, hydroxyalkyl cellulose, alkylhydroxyalkyl cellulose, cellulose sulfate, a salt of carboxymethyl cellulose, carboxymethyl cellulose or carboxyethyl cellulose.

7. The wound dressing of any one of claims 1 to 6 wherein said aldehyde-oxidized polysaccharide comprises aldehyde-oxidized polysaccharide comprising repeating units of Structure I 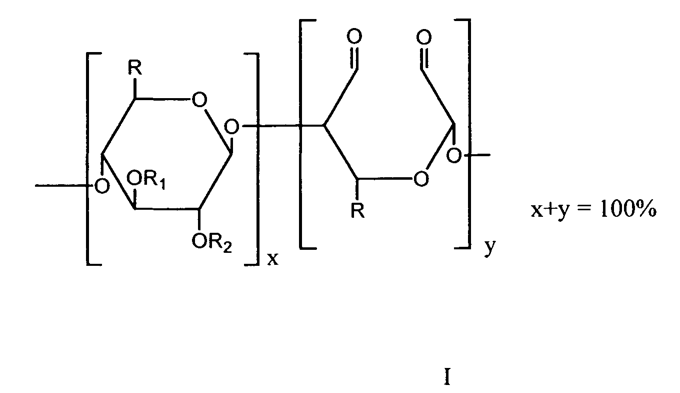 where x and y represent mole percent, x plus y equals 100 percent, x is from 95 to 5, y is from 5 to 95; and R may be CH₂OR₃, COOR₄, sulphonic acid or phosphonic acid; R may be H, alkyl, aryl, alkoxy or aryloxy, and R₁ and R₂ may be H, alkyl, aryl, alkoxy, aryloxy, sulphonyl or phosphoryl.

8. The wound dressing of claim 7 wherein said aldehyde-modified polysaccharide is prepared from hydroxyethyl cellulose or ethyl cellulose.

9. The wound dressing of any one of claims 1 to 8 wherein the weight ratio of said biodegradable water-soluble or water-swellable aldehyde-modified polysaccharide to said fabric is from 1:99 to 20:80.

10. The wound dressing of claim 8 or claim 9 wherein the weight ratio of said aldehye-oxidized polysaccharide to said fabric is from 3:97 to 10:90.
